# EUROPEAN PATENT SPECIFICATION

(11) **EP 3 723 663 B1**
(45) Date of publication and mention of the grant of the patent: **04.03.2026**
(21) Application number: 19702122.3
(22) Date of filing: 10.01.2019
(51) Int. Cl.: A61F 2/04, A61F 2/07

(54) **UROLOGICAL STENT**
UROLOGISCHER STENT
STENT UROLOGIQUE

(30) Priority: 16.01.2018 US 201862617721 P
(43) Date of publication of application: 21.10.2020
(73) Proprietor: Olympus Corporation, Hachioji-shi Tokyo 192-8507 (JP)
(72) Inventor: KOROSCHETZ, Moritz, Southborough, Massachusetts 01772 (US)
(74) Representative: Noack, Andreas
(86) International application number: PCT/IB2019/050193
(87) International publication number: WO 2019/142077

(56) References cited:
- EP-A1- 0 604 022
- EP-A1- 2 236 089
- WO-A1-2008/023160
- WO-A2-03/093338
- WO-A2-2007/022201
- CA-A1- 2 043 601
- US-A1- 2002 111 590
- US-A1- 2005 019 404
- US-A1- 2006 229 711
- US-A1- 2010 094 195
- US-B2- 7 261 734

## Description

### BACKGROUND

### Technical Field

The exemplary and non-limiting embodiments relate generally to urology and, more particularly, to a stent.

### Brief Description of Prior Developments

Ablative Benign Prostatic Hyperplasia (BPH) procedures involve trans-urethral removal of tissue which results in destruction of the urethra wall within the ablated prostate area. The destruction of this area causes swollen and irritated tissue and makes coagulation of opened blood vessels necessary. This is the origin for Post-operative bleeding, urinary irritation, urinary tract infections (UTI), bladder neck contractions (BNC) and obstruction.

In order to manage these post-operative complications, today's standard of care is the placement of a Foley bladder catheter. A 2-way or 3-way Foley catheter is placed in the patient after the ablative procedure. A 3-way Foley catheter is used in case of severe bleeding during the procedure in order to allow post-operative continuous bladder irrigation (CBI). Otherwise, patients may receive a 2-way Foley catheter. In case of continuous bladder irrigation (CBI), patients may be kept overnight in an observation area. Patients without a necessity for continuous bladder irrigation (CBI) may be released the same day of the procedure. Removal of a catheter from the patient is done either before the patient is released after an overnight stay or in an office visit to the treating urologist after approximately 48 hours of a patient's discharge with a 2-way catheter.

While the catheter helps to manage the potential side effects of the ablative procedure, the usage of a catheter is very uncomfortable for the patient and implies several risks and disadvantages such as discomfort, infection risk, unintended removal, etc. Available catheters address some of the issues encountered by using hydrophilic coatings and/or antibacterial silver-coatings, but do not change the basic concept of using a Foley catheter after an Ablative Benign Prostatic Hyperplasia (BPH) procedure. Document WO2007/022201 discloses an absorbable endo-urological composite tubular stent with an absorbable polymer spring and a water-swellable matrix, wherein more than 60% of the matrix cover an outer surface of the spring.

### SUMMARY

The following summary is merely intended to be exemplary. The summary is not intended to limit the scope of the claims, which define the present invention.

In accordance with one aspect, an example embodiment of the present invention is provided in an apparatus comprising a first layer, where the first layer comprises a scaffold structure forming an inner lumen along a length of the scaffold structure, and where the first layer comprises a bioresorbable material; and a second layer on the first layer, where the second layer comprises a bioresorbable material, where the second layer surrounds a majority of the first layer, and where the second layer is configured to hydroscopicly swell. The scaffold structure has a distal end which extends past a distal end of the second layer and comprises protruding anchors which extend into the second layer and are sized and shaped to fix the scaffold structure to tissue of a patient after the second layer is resorbed or dissolved inside the patient.

In accordance with another aspect, an example embodiment not part of the invention is provided in an apparatus comprising a first layer, where the first layer comprises a scaffold structure forming an inner lumen along a length of the scaffold structure, and where the first layer comprises a bioresorbable material; and a second layer on the first layer, where the second layer comprises a bioresorbable material, where the second layer surrounds a majority of the first layer, and where the first layer has a first degradation rate which is different than a second degradation rate of the second layer.

In accordance with another aspect, an example embodiment not part of the invention is provided in an apparatus comprising a first member, where the first member comprises an inner lumen along a length of the first member, and where the first member comprises a bioresorbable material; and a second member on the first member, where the second member comprises a bioresorbable material, where the second member surrounds at least a portion of the first member, where the second member is compressible on the first member, and where the second member is configured to resiliently expand on the first member from a compressed configuration relative to the first member to an expanded configuration relative to the first member.

In accordance with another aspect, an example method according to the invention comprises providing a first member, where the first member comprises a scaffold structure forming an inner lumen along a length of the scaffold structure, and where the first member comprises a bioresorbable material; providing a second member on the first member, where the second member comprises a bioresorbable material, where the second layer surrounds at least a portion of the first member, and where the second member is configured to hydroscopicly swell.

In accordance with another aspect not part of the invention, an example method comprises providing a first layer, where the first layer comprises a structure forming an inner lumen along a length of the structure, and where the first layer comprises a bioresorbable material; providing a second layer on the first layer, where the second layer comprises a bioresorbable material, where the second layer surrounds at least a portion of the first layer, and where the first layer has a first degradation rate which is different than a second degradation rate of the second layer.

In accordance with another aspect, an example method not part of the invention comprises providing a first member, where the first member comprises an inner lumen along a length of the first member, and where the first member comprises a bioresorbable material; providing a second member on the first member, where the second member comprises a bioresorbable material, where the second member surrounds at least a portion of the first member, where the second member is compressible on the first member, and where the second member is configured to resiliently expand on the first member from a compressed configuration relative to the first member to an expanded configuration relative to the first member.

In accordance with another aspect not part of the invention, an example method comprises inserting an apparatus into a prostatic urethra of a patient, where the apparatus comprises a first member and a second member surrounding at least a portion of the first member, where the first member comprising a scaffold structure forming an inner lumen along a length of the scaffold structure, where the first member comprises a bioresorbable material, where the second member comprises a bioresorbable material, and where the second member is configured to hydroscopicly swell; and exposing the apparatus to liquid while in the prostatic urethra of the patient to cause the second member to swell and press against an inner surface of the prostatic urethra.

In accordance with another aspect not part of the invention, an example method comprises inserting an apparatus into a prostatic urethra of a patient, where the apparatus comprises a first member and a second member on the first member, where the first member comprises an inner lumen along a length of the first member, where the first member comprises a bioresorbable material, where the second member surrounds at least a portion of the first member, where the second member is compressible on the first member, where the second member is configured to resiliently expand on the first member from a compressed configuration relative to the first member to an expanded configuration relative to the first member, where the second member is in the compressed configuration when the apparatus is inserted into the prostatic urethra of the patient; and allowing the second member to expand relative to the second member after the apparatus is inserted into the prostatic urethra of the patient, where an outer surface of the second member presses against an inner surface of the prostatic urethra as the second member expands.

### BRIEF DESCRIPTION OF THE DRAWINGS

The foregoing aspects and other features are explained in the following description, taken in connection with the accompanying drawings, wherein:
Fig. 1 is a diagram illustrating a portion of male human anatomy;
Fig. 2 is a side view of an example embodiment;
Fig. 3 is a perspective view of the example embodiment shown in Fig. 2 with an enlargement of a distal end;
Fig. 4 is a cross sectional view of the example embodiment shown in Figs. 2-3;
Fig. 5 is a perspective view of the example embodiment shown in Figs. 2-4 in an expanded configuration;
Fig. 6 is a side view of the example embodiment shown in Fig. 5;
Fig. 7 is a diagram illustrating placement of the example embodiment shown in Figs. 2-6 in the prostate and bladder of a patient;
Figs. 8-10 illustrate an example method of placing the example embodiment shown in Figs. 2-6 into the patient;
Fig. 11 is a perspective of an alternate example embodiment;
Fig. 12 is a side view of the example embodiment shown in Fig. 11 in an expanded configuration;
Fig. 13 is a cross sectional view of the example embodiment shown in Fig. 12;
Fig. 14 is a perspective of an alternate example embodiment;
Fig. 15 is a side view of the example embodiment shown in Fig. 14;
Fig. 16 is a cross sectional view of the example embodiment shown in Figs. 14-15;
Fig. 17 is a partial perspective of the first member of any one of the example embodiments showing the anchors;
Figs. 18-22 illustrate an example method of placing an example embodiment;
Fig. 23 illustrates an example insertion apparatus;
Figs. 25-27 illustrate some examples of instruments which can be used in positioning any one of the example embodiments;
Fig. 28 is a diagram illustrating an example method;
Fig. 29 is a diagram illustrating an example method;
Fig. 30 is a diagram illustrating an example method.

### DETAILED DESCRIPTION OF EMBODIMENTS

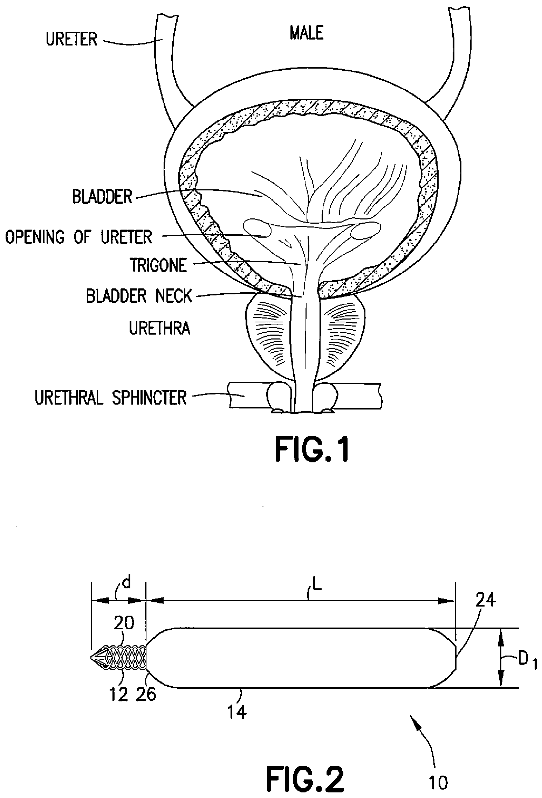

Fig. 1 is a diagram illustrating some features of male human anatomy. In particular, a bladder is shown. The urethra extends from the bladder including the prostatic urethra through the prostate. The locations of the bladder neck and the urethral sphincter are also shown.

Referring also to Fig. 2, there is shown a side view of an example embodiment of an apparatus 10 incorporating features as described below. Although the features will be described with reference to the example embodiments shown in the drawings, it should be understood that features can be embodied in many alternate forms of embodiments. In addition, any suitable size, shape or type of elements or materials could be used.

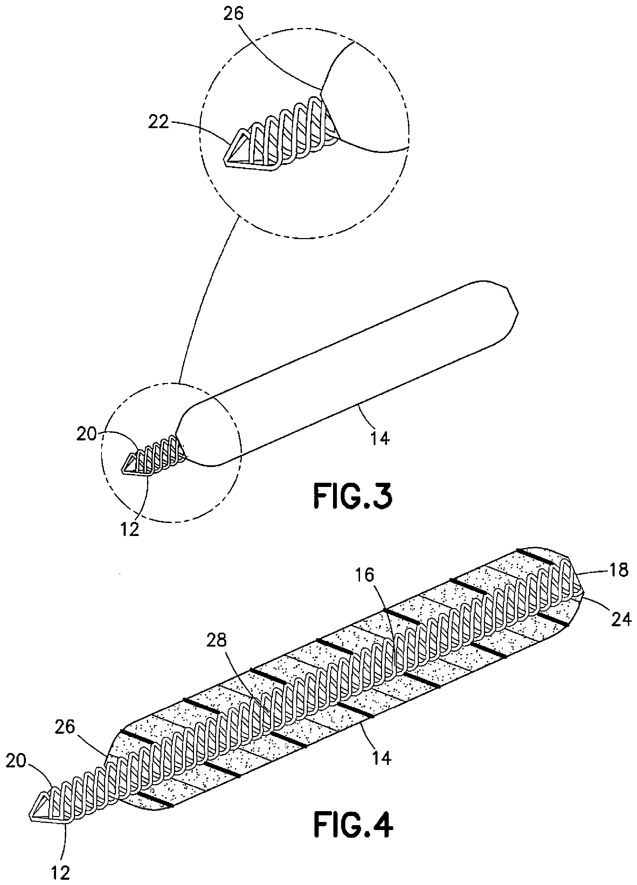

The apparatus 10 in this example is a stent. More particularly, in this example embodiment the apparatus 10 is a urological stent configured to be inserted into the urethra of a patient. Referring also to Figs. 3-4, the stent 10 comprises a first member 12 forming a first layer and a second member 14 forming a second layer. The second member 14 is either formed on the first member 12 or attached to the first member to form the stent as a unitary structure for insertion into the patient's urethra.

The first member 12 has a general tubular shape forming an inner lumen 16 between a proximal end 18 and a distal end 20. The first member 12, in this example embodiment, has a general scaffold structure formed as a lattice or grille. In this example the proximal end 18 of the first member 12 is open, and the distal end 20 of the first member 12 has the lattice or grille at its tip 22 as shown best in the enlarged portion of Fig. 3. The proximal end 18 is substantially flush with the proximal end 24 of the second member 14. The distal end 20 of the first member 12 extends outward from the distal end 26 of the second member 14. However, in alternate embodiments the distal end of the first member 12 might be substantially flush with the distal end of the second member 14, and/or the proximal end of the first member 12 might extend outward from the proximal end 24 of the second member 14. The first member 12 in this example embodiment is comprised of at least one bioresorbable material such as, for example, poly(L-lactide) or poly(D,L-lactide). The scaffold structure may be a flexible scaffold and is configured to provide a structural strength and sufficient radial force to hold the urethra open and, thus, allow passage of urine through the inner lumen from the distal end 20 to the proximal end 18. In one type of example the scaffold structure may be provided as initially in a radially collapsed configuration about the longitudinal axis of the inner lumen, at least partially, which is subsequently allowed to expand or deploy, such as by self-expandable resilient deflection, into an expanded configuration. The first member 12 may comprise outward extending barbs or anchors at the section 28 inside the second member 14 and/or at the distal end 20 which is not covered by the second section 14.

The second member 14 forms a second layer around at least a portion of the first layer formed by the first member 12. The second member 14 also comprises at least one bioresorbable material which may be at least partially the same as the bioresorbable material(s) of the first member. In this example embodiment the second member is formed as a chitosan sponge-type outer layer which uses the properties of chitosan to support healing of treated mucosa. Chitosan is a linear polysaccharide composed of randomly distributed β-(1→4)-linked D-glucosamine (deacetylated unit) and N-acetyl-D-glucosamine (acetylated unit). In medicine, it is useful in bandages to reduce bleeding and as an antibacterial agent. Chitosan's properties allow it to rapidly clot blood.

An example of some chitosan material is described in International Publication Number WO 2016/178829 A1, having a U.S. equivalent in U.S. application No. 15/571,681. For example, the following U.S. patents are mentioned in WO 2016/178829 A1: 8, 709, 463; 8,414,925; 7,279,177; 7,019,191; 6,060,461; and publication No. 2007/0087061.

Although the second member 14 is described as forming a second layer of the stent 10, it should be understood that the second member 14 may comprise more than one layer or portion of bioresorbable material (s). In the example embodiment shown, the first member has a dissolution rate or degradation rate which is slower than a dissolution rate or degradation rate of the second member. The second member 14 may have a general resilient-type characteristics such as being sponge-like porous such as being configured to hydroscopicly swell, and/or being resiliently deflectable or compressible. The second member is configured to provide a first compressed or collapsed configuration and a second expanded configuration as further understood from the description below.

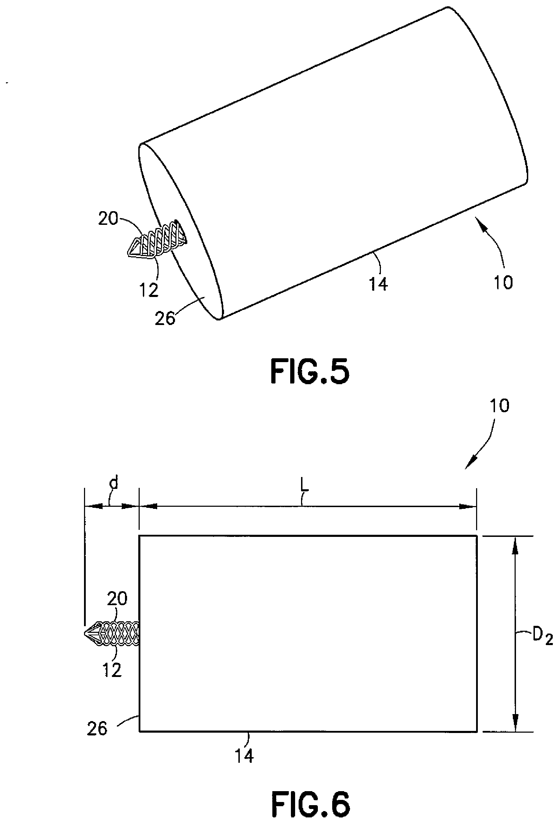

Figs. 2-3 show the second member 14 in its first compressed or collapsed configuration relative to the first member 12. Referring also to Figs. 5-6, the second member 14 is shown in its second expanded configuration relative to the first member 12. In this example the second member 14 has hydroscopicly swelled on the first member 12. The sponge type outer layer 14 could be compressed during a production process for easier insertion into the patient and be expanded after initial insertion by irrigation during insertion process. As the second member 14 hydroscopicly swells, it expands radially outward relative to the longitudinal axis of the inner lumen of the first member 12. In this second expanded configuration the outer diameter D₂ of the second member 14 is enlarged relative to the outer diameter D₁ of the second member 14 in its first compressed or collapsed configuration shown in Fig. 2. In one type of example embodiment the second member 14 is configured to expand about 200-500 percent versus its first compressed or collapsed configuration. However, in alternate example embodiments the second member 14 may be configured to expand less than 200 percent. In another alternate example embodiment the second member 14 may be configured to expand more than 500 percent. The first member 12 is configured to structurally keep the inner lumen 16 open even when the second member 14 expands into its second expanded configuration, such as through hydroscopic swelling and/or other expansion. Thus, even in the second expanded configuration shown in Figs. 5-6 fluid can still flow through the stent 10 via the inner lumen 16.

In this example embodiment the length L of the second member 14 remains substantially unchanged between the first compressed or collapsed configuration shown in Fig. 2 and the second expanded configuration shown in Fig. 6. Likewise, the distal end 20 of the first member 12 also still extends or projects outward from the distal end of the second member 14 with the distance d of projection from the distal end 26 remaining substantially unchanged.

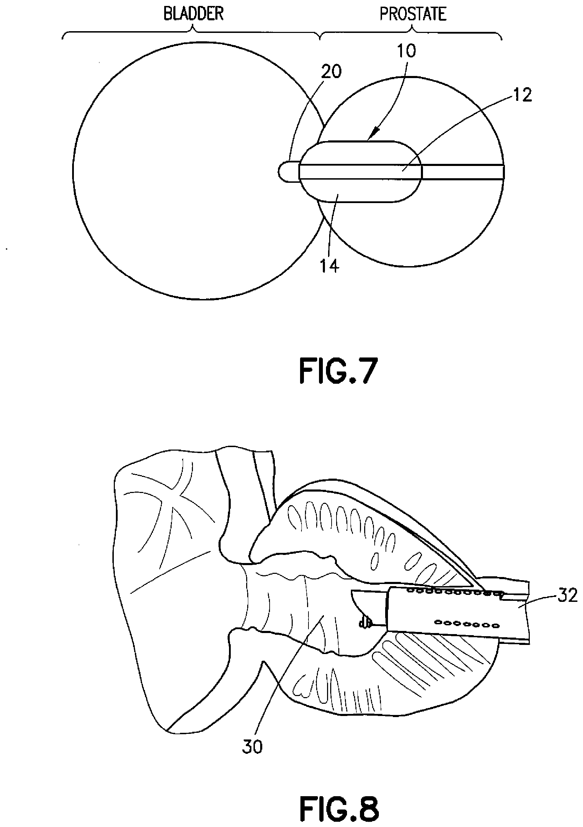

Referring also to Fig. 7, the stent 10 is configured to be inserted into a patient's prostate along the urethra and, at least partially, extend into the patient's bladder. As shown, the distal end 20 of the first member provides a bladder protruding tip preventing potential debris from clogging the lumen 16. The hemostatic material (such as chitosan) expands into a volume created by a procedure, such as an ablative Benign Prostatic Hyperplasia (BPH) procedure for example, to insure contact with the tissue in the prostate. The first member 12 provides a structure to an open lumen allowing evacuation of urine from the bladder.

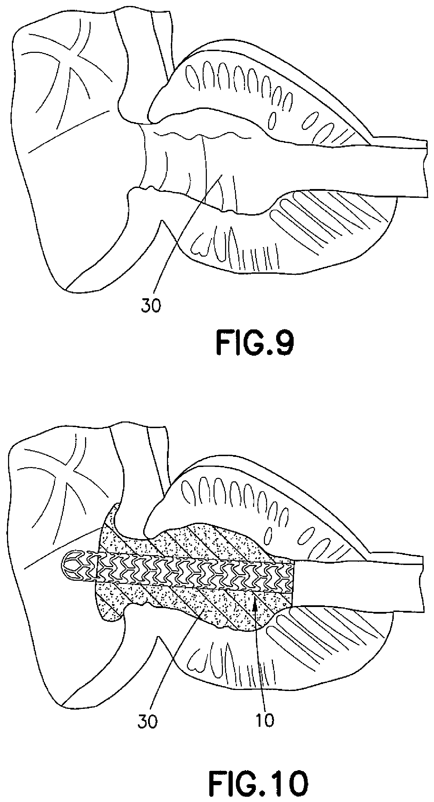

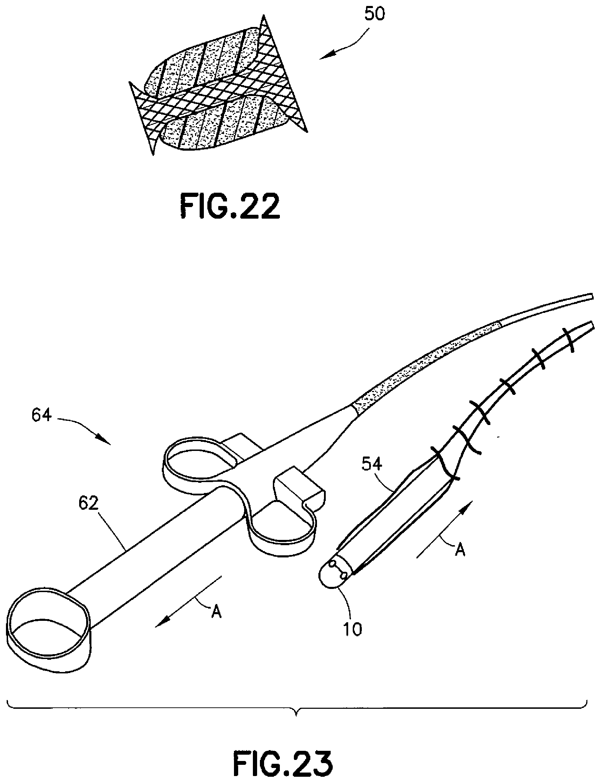

Referring also to Figs. 8-10, one example of using the stent 10 will be described. As illustrated by Figs. 8-9, a user may use an instrument 32, such as a resectoscope for example, for an ablative Benign Prostatic Hyperplasia (BPH) procedure on the patient's prostate, such as laser therapy for example. This causes an enlarged open area 30 to be formed in the prostate. If necessary, and if not already determined by previous diagnostics, the user may measure the distance from bladder neck to verumontanum with the resectoscope to choose a suitable length of the prostatic chitosan stent 10 from a plurality of different length stents 10. Once the enlarged open area 30 is formed (after the resection) the user may remove the inner sheath, the telescope and working element of the resectoscope and leave the outer sheath of the resectoscope in place in the patient's urethra. The user may then insert the stent 10 into the patient's prostate, through the outer sheath of the resectoscope, and allow or cause the second member 14 to expand into a seating contact with the prostate and bladder neck. The insertion may comprise inserting a prostatic chitosan stent with one hand operated stent introducer (such as shown in Fig. 23 for example) and push the stent into the bladder. Positioning of the prostatic chitosan stent in the right position may be accomplished by using units on an introducer. As shown in Fig. 23 for example, the introducer may then be used to remove a protective film from the implant/stent by pulling on a portion of the introducer. This allows or causes the second member 14 to expand to help fill the open area 30 as shown in Fig. 10 and, such as by hydroscopic swelling for example. Continuous irrigation can be used until full expansion of prostatic chitosan stent.

With features as described herein, the prostatic urethra may be held open after a procedure because of the radial force delivered by the inner scaffold structure. Urine from the bladder may be evacuated through the inner lumen 16, and activity of the external sphincter is not affected after the placement of the stent. The chitosan sponge layer is able to expand against the mucosa at the prostate in order to support hemostasis and healing of the urethral epithelium, and the muco-adhesive properties help to prevent migration of the implanted stent 10.

In one example embodiment, where the first and second layers of the stent may be configured to be resorbed at different rates. The sponge type chitosan outer layer may be configured to be absorbed in a first period of approximately 24-48 hours for example. The inner scaffold structure may be configured to be resorbed after this first period within a second period of about 1 week for example; thus preventing flow obstruction over the whole healing period of the epithelium. Please note that these periods of time are merely examples and should not be considered as limiting. Longer dissolution rates may not be suitable in order to prevent encrusting of scaffold structure.

Anchors in the scaffold structure may be used to prevent migration of the scaffold structure once the chitosan layer has dissolved and during the subsequent period of time it takes for the complete dissolution of the inner structure.

With the features described herein, the stent may be used to lower a patient's pain score by using the stent as a soft, spongy implant to replace use of a catheter. This can also improved the quality of life of the patient, after an ablative Benign Prostatic Hyperplasia (BPH) procedure for example, because of the absence or elimination of use of a catheter and urine bag; allowing the patent to obtain a quicker return to his daily routine. The stent will also help to reduce a risk of post-operative bleeding due to the hemostatic properties of chitosan in the second member 14. In addition, with use of the stent 10, there will be no need for the patient to go to an additional office/hospital visit for removal of a catheter because a catheter is no longer needed after the ablative Benign Prostatic Hyperplasia (BPH) procedure and because of the stent's use of bioresorbable material. These features may be obtained with use of a potentially self-expandable stent made of two bioresorbable layers; an inner structure, such as a scaffold structure for example, surrounded by a chitosan layer that supports hemostasis and healing of the urethra and holds the urethra open bioresorbable.

In some example embodiments, features may include, for example:
- a diameter extension of the chitosan sponge layer serving as a bladder neck covering shape;
- anchors on the scaffold structure preventing migration of the remaining scaffold after resorption of the outer chitosan layer;
- the bladder protruding scaffold tip 22 allowing evacuation of urine and preventing obstruction of the inner lumen by remaining tissue in the bladder or a protruding tip (intended to protrude into the bladder) which does not comprises the scaffold shaped tip.

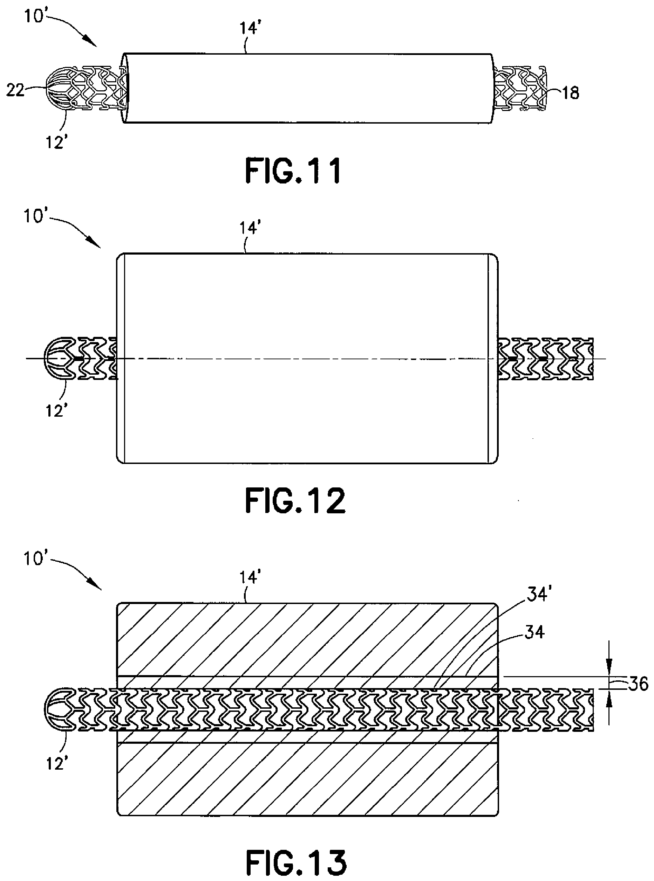

Referring also to Fig. 11, another example embodiment is illustrated. In this example the stent 10' is shown in its non-expanded shape, and comprises the first member 12' and the second member 14' which are substantially identical to the stent 10 described above, but with differences as noted in the following. The distal tip 22 of the first member 12' has a more rounded shape. The proximal end 18 of the first member 12' extends outward from the proximal end of the second member 14'. The distal end and the proximal end of the second member 14' have substantially flat shapes rather than the rounded shapes of the second member 14.

Referring also to Figs. 12-13 the stent 10' is shown in its expanded configuration. In particular, the second member 14' has expanded, such as due to hydroscopic swelling for example. As illustrated in Fig. 13, the initial inner diameter 34 of the second member 14' has swelled inward to increase contact with the first member 12' at diameter 34'. Before this swelling, the second member 14' might merely be attached to the first member 12' by tips of outward protruding barbs or anchors of the first member 12'. The gap 36 may help to insure that the first member 12' is not collapsed by the second member 14' as the second member 14' swells.

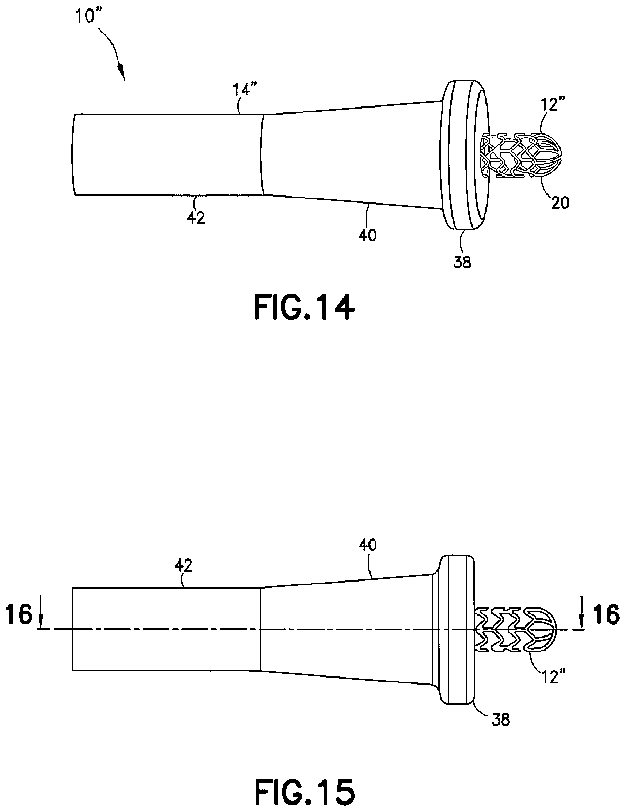

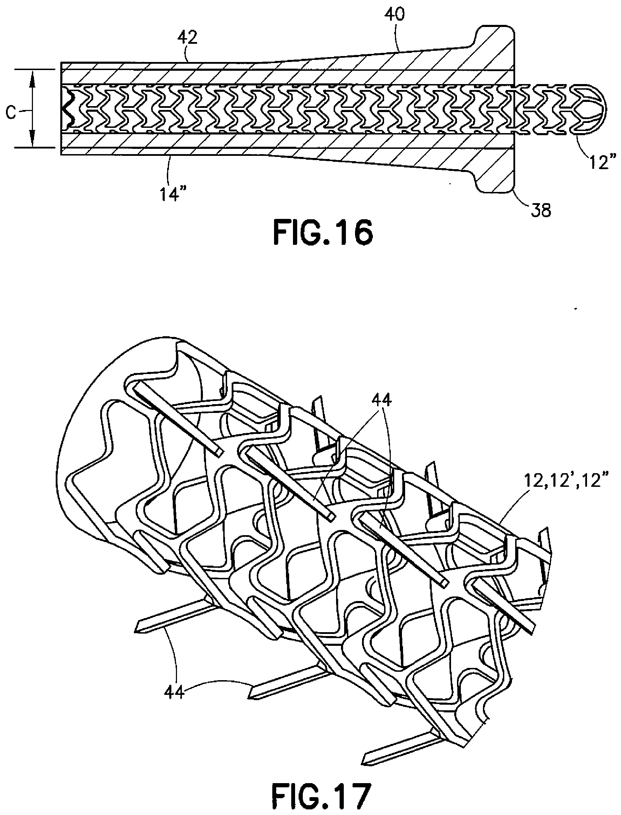

Referring also to Figs. 14-16, another example embodiment is shown. Figs. 14-16 show a stent 10" in its expanded configuration. In this example the stent 10' comprises the first member 12" and the second member 14" which are substantially identical to the stents 10 and 10' described above, but with differences as noted in the following. The stent 10" in its non-expanded configuration may look similar to the stent 10' shown in Fig. 11, but without the proximal end of the first member 12" extending out of the proximal end of the second member 14". The second member 14" has a substantially uniform outer diameter in the non-expanded configuration as illustrated by diameter C shown in Fig. 16, but expands into a non-uniform outer diameter as shown in Figs. 14-16 as the second member 14 expands, such as by hydroscopic swelling for example, and/or by means of removing a compression covering. In this example, the non-uniform exterior shape of the second member 14" in its expanded configuration includes a distal section 38, a proximal section 42 and a middle section 40. The distal section 38 provides an enlarged section which is configured to be located inside the bladder and function as a bladder neck cover to seat against the bladder neck of the patient. This can help the user to position the stent 10" and prevent the stent 10" from moving away from the bladder neck; at least until dissolved. The middle section 40 can have a general conical shape extending down to the narrower diameter proximal section 42. The middle section 40 and the proximal section 42 can form the active length in the bladder. In one type of example, the shape of the middle section 40 and the proximal section 42 may be designed based upon clinical observations of the general shape of the area 30 usually formed by resection to thereby fit or fill the area 30 efficiently as illustrated best by Fig. 10; perhaps with more uniform contact force or pressure against all areas of the resection.

Referring also to Fig. 17, one example of anchors or barbs 44 on the first member 12, 12' or 12" is shown. The anchors 44 may be shaped to point in both proximal and distal directions at angles, such as shown in this example embodiment.

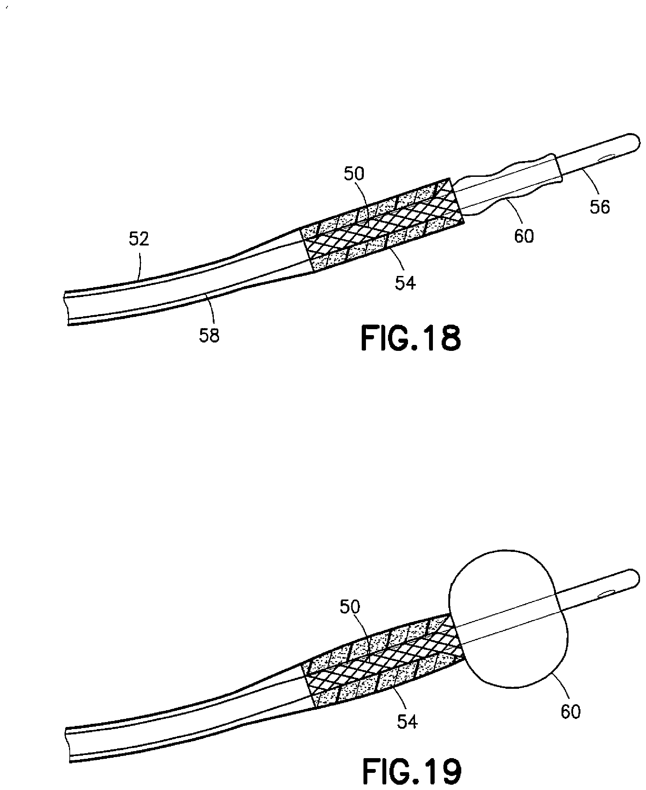

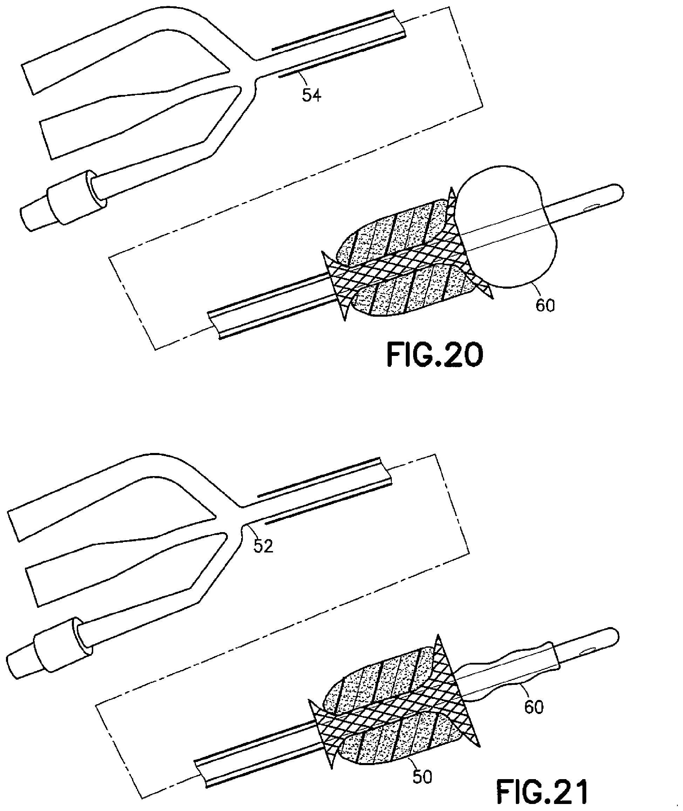

Referring also to Figs. 18-22, one example method of placing a stent comprises features as described herein will be described. In this example the stent 50, comprising a compressed layer of chitosan and bioresorbable stent structure is placed on the distal end of a catheter 52 such as a two-way or three-way Foley catheter for example. The chitosan layer is compressed and protected by a retractable protective film layer or cover 54 as shown in Fig 18. In this example, the distal end 56 of the catheter 52 has a smaller thickness/diameter than the rest of the catheter shaft 58. This provides a seat for the stent 50 without significantly enlarging the diameter of the assembly shown in Fig. 18. The catheter 52 has a balloon section 60. After the assembly is located at the prostate, the balloon section 60 may be inflated as shown by Fig. 19 to help position and hold the stent relative to the prostate. The retractable protective film layer or cover 54 may then be retracted as indicated by Fig. 20 to allow the stent 50 to expand inside the patient. This expansion may be merely resilient expansion and/or hydroscopic expansion of the second layer of the stent. In this example the first member or braid has a funnel shaped proximal end when allowed to expand. The braid is longer than the chitosan layer on the distal end and has the funnel shape to anchor between the verumontanum and the external urethral sphincter. As illustrated by Fig. 21, the balloon 60 may be deflated and the catheter 52 removed from the stent 50 to leave the stent 50 deployed in the patient's prostate as shown in Fig. 22.

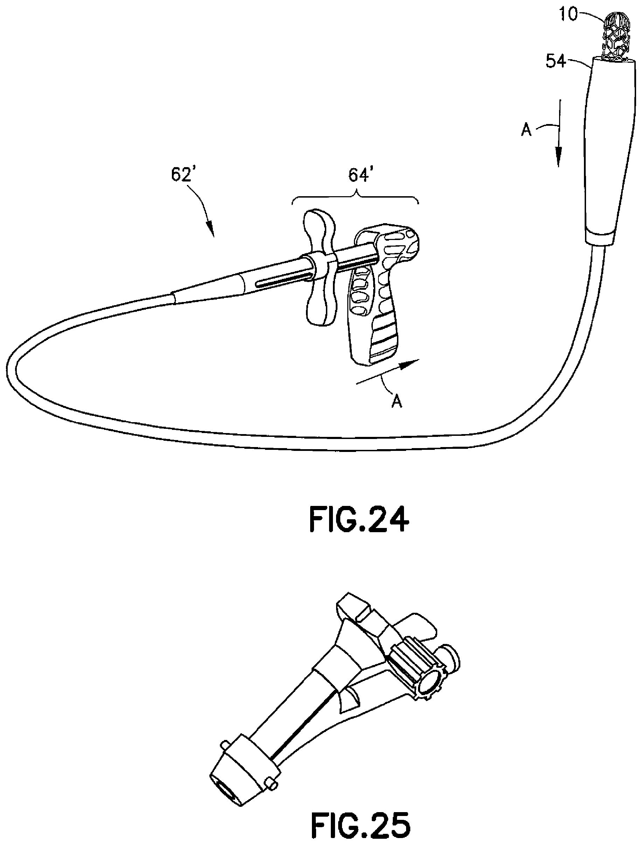

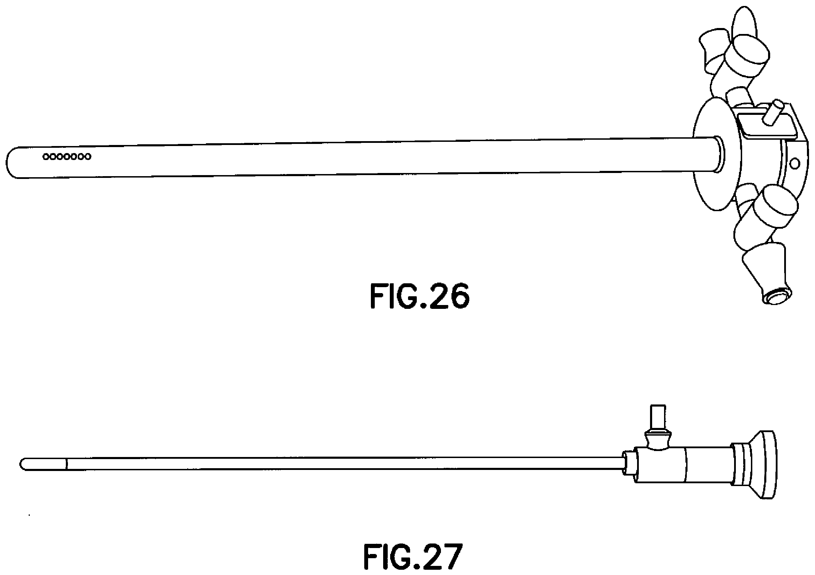

Referring also to Fig. 23 an instrument 62 is shown which may be used in order to introduce the chitosan-stent as a one-hand operated stent delivery system. Another example of an instrument 62' as a one-hand operated stent delivery system is shown in Fig. 24. The handles 64, 64' of the instruments 62, 62' are configured to slide the protective/compression covers 54 off of the outer layers of the stents 10 as indicated by arrows A, illustrating motion of portions of the handles and the cover 54, after the stents are located at the prostate. The delivery systems/instruments could be used, for example, in combination with stent delivery bridge as shown in Fig. 24, or a resectoscope outer sheath as shown in Fig. 26, or a telescope as shown in Fig. 27.

Ablative BPH procedures involve trans-urethral removal of tissue which results in destruction of the urethra wall within the ablated prostate area. The destruction of this area causes swollen and irritated tissue and makes coagulation of opened blood vessels necessary. This can cause post-operative bleeding, urinary irritation, urinary tract infections (UTI), bladder neck contractions (BNC) and/or obstruction. Other more minimally invasive procedures (Rezum & PUL) also lead to irritated urethral mucosa, swollen prostate tissue and/or open blood vessels which can lead to post-operative bleeding, urinary irritation, urinary tract infections (UTI) and/or obstruction. Today's standard of care is the placement of a Foley bladder catheter in all of the cases, which can be very uncomfortable for the patient and implies several risks and disadvantages such as discomfort, infection risk, unintended removal etc. The prostatic chitosan stent described herein provides an improved post operative care through its hemostatic properties, its healing supporting properties while maintaining voluntary urination without need of removal.

Features as described herein provide a way to improve post operative healing of the urethra. This may be provided with a dissolvable prostatic chitosan stent with a braided inner structure and chitosan (sponge type) outer layer. The dissolvable prostatic chitosan stent may be used to provide an improved post operative care after a transurethral procedure for BPH by supporting hemostasis and healing process of urethra in general. This may be used to replace the current standard of care leaving a Foley catheter in the patient after completion of the procedure.

The dissolvable prostatic chitosan stent, placed in the prostatic urethra, may be used to hold the prostatic urethra open and, thus, allow voluntary drainage of the bladder by the patient. This may be used to maintain an appropriate opening of the prostatic urethral lumen for voluntary drainage of the bladder. The stent may also perform a hemostatic action, and may dissolves within about 14 days for example. This may be used to improve patient quality of life compared to use of a Foley catheter, reducing post-operative bleeding by performing hemostatic action, and reduce post-operative urinary track infections by providing a bacterial barrier (zone of inhibition).

The exterior chitosan material (such as primarily Carboxymethyl Chitosan mixed with cellulose for example) may be used to form an absorbent porous sponge that is flexible and dissolvable. Once irrigated, this chitosan sponge will expand and press against the urethral mucosa. Because of the properties of chitosan, it will act as a hemostat and as an adjunct to aid in the natural healing process of the urethral epithelium. The braided inner structure will provide an open lumen for evacuation of urine. The stent may be placed proximal to the external sphincter and does not affect its function. The stent may be made available in different lengths in order to fit different prostate lengths.

In one example embodiment, a medical device may be provided comprising a bio-absorbable outer layer including a lumen having a distal end and a proximal end, the distal end having a different diameter than the proximal end; and bio-absorbable porous inner layer positioned partially in the outer layer.

In one example embodiment an apparatus is provided comprising a first layer, where the first layer comprises a scaffold structure forming an inner lumen along a length of the scaffold structure, and where the first layer comprises a bioresorbable material; and a second layer on the first layer, where the second layer comprises a bioresorbable material, where the second layer surrounds a majority of the first layer, and where the second layer is configured to hydroscopicly swell.

The scaffold structure may have a general lattice shape may include at a tip of a distal end of the scaffold structure. The distal end of the scaffold structure may extend past a distal end of the second layer. The scaffold structure may have a distal end which extends past a distal end of the second layer. The scaffold structure may comprise protruding anchors which extend into the second layer. The protruding anchors may be sized and shaped to fix the scaffold structure to tissue of a patient after the second layer is resorbed or dissolved inside the patient. At least one portion of the scaffold structure may be configured to expand from a collapsed configuration to an expanded configuration inside a patient. The second layer may be configured to hydroscopicly swell into a shape having a enlarged distal end configured to be located against a bladder neck of a patient. The second layer may be configured to hydroscopicly swell into the shape with an outer conical shape extending from the enlarged distal end in a direction towards a proximal end of the second layer. At least one portion of the second layer may have an outer diameter which is configured to hydroscopicly swell between about 200-500 percent from a non-swelled first configured to a hydroscopicly swelled second configuration. The apparatus may further comprise a protective cover on the second layer to limit moisture entering the second layer, where the protective cover is configured to be slid off of the second layer after the apparatus is inserted into patient. The first layer may have a first degradation rate which is different than a second degradation rate of the second layer. The first layer may comprise a first member, where the second layer comprises a second member, where the second member is compressible on the first member, and where the second member is configured to resiliently expand on the first member from a compressed configuration relative to the first member to an expanded configuration relative to the first member. The apparatus may be configured to be inserted into a prostatic urethra of a patient, where the second layer is configured to expand relative to the first layer after the apparatus is inserted into the prostatic urethra of the patient, and where an outer surface of the second layer is configured to press against an inner surface of the prostatic urethra as the second member expands.

In one example embodiment an apparatus may be provided comprising a first layer, where the first layer comprises a scaffold structure forming an inner lumen along a length of the scaffold structure, and where the first layer comprises a bioresorbable material; and a second layer on the first layer, where the second layer comprises a bioresorbable material, where the second layer surrounds a majority of the first layer, and where the first layer has a first degradation rate which is different than a second degradation rate of the second layer.

The bioresorbable material of the second layer may be at least partially different from the bioresorbable material of the first layer. The second layer may be configured to hydroscopticly swell. The second degradation rate may be configured to allow the second layer to be dissolved or resorbed in a patient between about 18-60 hours and the first degradation rate allows the first layer to be dissolved or resorbed in the patent between about 10-30 days. The scaffold structure may have a general tubular lattice shape including a lattice shape at a tip of a distal end of the scaffold structure. The distal end of the scaffold structure may extend past a distal end of the second layer. The scaffold structure may have a distal end which extends past a distal end of the second layer. The scaffold structure may comprise protruding anchors which extend into the second layer. The protruding anchors may be sized and shaped to fix the scaffold structure to tissue of a patient after the second layer is resorbed or dissolved inside the patient. At least one portion of the scaffold structure may be configured to expand from a collapsed configuration to an expanded configuration inside a patient. The second layer may be configured to hydroscopicly swell into a shape having a enlarged distal end configured to be located against a bladder neck of a patient. The second layer may be configured to hydroscopicly swell into the shape with an outer conical shape extending from the enlarged distal end in a direction towards a proximal end of the second layer. At least one portion of the second layer may have an outer diameter which is configured to hydroscopicly swell between about 200-500 percent from a non-swelled first configured to a hydroscopicly swelled second configuration. The apparatus may further comprise a protective cover on the second layer to limit moisture entering the second layer, where the protective cover is configured to be slid off of the second layer after the apparatus is inserted into patient. The first layer may comprise a first member, where the second layer may comprise a second member, where the second member is compressible on the first member, and where the second member is configured to resiliently expand on the first member from a compressed configuration relative to the first member to an expanded configuration relative to the first member. The apparatus may be configured to be inserted into a prostatic urethra of a patient, where the second layer is configured to expand relative to the first layer after the apparatus is inserted into the prostatic urethra of the patient, and where an outer surface of the second layer is configured to press against an inner surface of the prostatic urethra as the second member expands.

In one example embodiment an apparatus may be provided comprising a first member, where the first member comprises an inner lumen along a length of the first member, and where the first member comprises a bioresorbable material; and a second member on the first member, where the second member comprises a bioresorbable material, where the second member surrounds at least a portion of the first member, where the second member is compressible on the first member, and where the second member is configured to resiliently expand on the first member from a compressed configuration relative to the first member to an expanded configuration relative to the first member.

The first member may have a first degradation rate which is different than a second degradation rate of the second member. The bioresorbable material of the second member may be at least partially different from the bioresorbable material of the first member. The second member may be configured to hydroscopticly swell and the first member is configured not to hydroscopticly swell. The second degradation rate may allow the second member to be dissolved or resorbed in a patient between about 18-60 hours and the first degradation rate may allow the first member to be dissolved or resorbed in the patent between about 10-30 days. The first member may comprise a scaffold structure with a general tubular lattice shape including a lattice shape at a tip of a distal end of the scaffold structure. The distal end of the scaffold structure may extend past a distal end of the second member. The first member may comprise a scaffold structure which has a distal end which extends past a distal end of the second member. The first member may comprise a scaffold structure which comprises protruding anchors which extend into the second member. The protruding anchors may be sized and shaped to fix the scaffold structure to tissue of a patient after the second member is resorbed or dissolved inside the patient. The first member may comprise a scaffold structure with at least one portion of the scaffold structure configured to expand from a collapsed configuration to an expanded configuration inside a patient. The second member may be configured to hydroscopicly swell into a shape having a enlarged distal end configured to be located against a bladder neck of a patient. The second member may be configured to hydroscopicly swell into the shape with an outer conical shape extending from the enlarged distal end in a direction towards a proximal end of the second member. At least one portion of the second member may have an outer diameter which is configured to hydroscopicly swell between about 200-500 percent from a non-swelled first configured to a hydroscopicly swelled second configuration. The apparatus may further comprise a protective cover on the second member to limit moisture entering the second member, where the protective cover is configured to be slid off of the second member after the apparatus is inserted into patient. The apparatus may be configured to be inserted into a prostatic urethra of a patient, where the second member is configured to expand relative to the first member after the apparatus is inserted into the prostatic urethra of the patient, and where an outer surface of the second member is configured to press against an inner surface of the prostatic urethra as the second member expands.

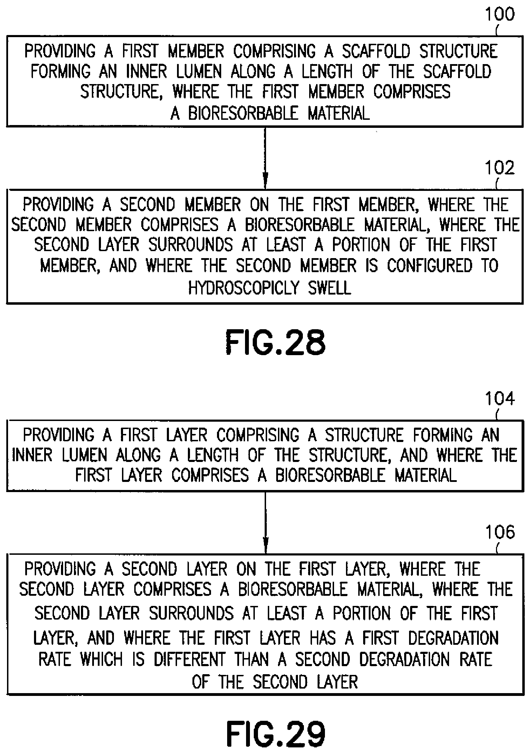

Referring also to Fig. 28, an example method may comprise providing a first member, where the first member comprises a scaffold structure forming an inner lumen along a length of the scaffold structure, and where the first member comprises a bioresorbable material as illustrated by block 100; providing a second member on the first member, where the second member comprises a bioresorbable material, where the second layer surrounds at least a portion of the first member, and where the second member is configured to hydroscopicly swell as illustrated by block 102.

Referring also to Fig. 29, an example method may comprise providing a first layer, where the first layer comprises a structure forming an inner lumen along a length of the structure, and where the first layer comprises a bioresorbable material as illustrated by block 104; providing a second layer on the first layer, where the second layer comprises a bioresorbable material, where the second layer surrounds at least a portion of the first layer, and where the first layer has a first degradation rate which is different than a second degradation rate of the second layer as illustrated by block 106.

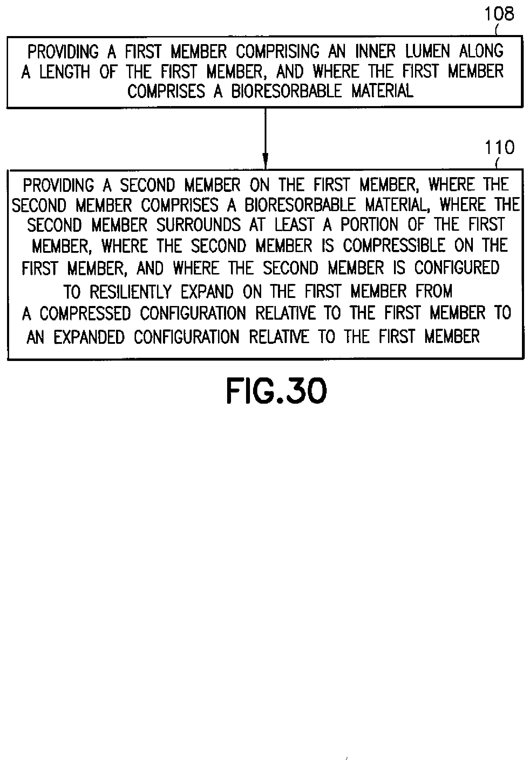

Referring also to Fig. 30, an example method may comprise providing a first member, where the first member comprises an inner lumen along a length of the first member, and where the first member comprises a bioresorbable material as illustrated by block 108; providing a second member on the first member as illustrated by block 110, where the second member comprises a bioresorbable material, where the second member surrounds at least a portion of the first member, where the second member is compressible on the first member, and where the second member is configured to resiliently expand on the first member from a compressed configuration relative to the first member to an expanded configuration relative to the first member.

An example method may comprise inserting an apparatus into a prostatic urethra of a patient, where the apparatus comprises a first member and a second member surrounding at least a portion of the first member, where the first member comprising a scaffold structure forming an inner lumen along a length of the scaffold structure, where the first member comprises a bioresorbable material, where the second member comprises a bioresorbable material, and where the second member is configured to hydroscopicly swell; and exposing the apparatus to liquid while in the prostatic urethra of the patient to cause the second member to swell and press against an inner surface of the prostatic urethra.

An example method may comprise inserting an apparatus into a prostatic urethra of a patient, where the apparatus comprises a first member and a second member on the first member, where the first member comprises an inner lumen along a length of the first member, where the first member comprises a bioresorbable material, where the second member surrounds at least a portion of the first member, where the second member is compressible on the first member, where the second member is configured to resiliently expand on the first member from a compressed configuration relative to the first member to an expanded configuration relative to the first member, where the second member is in the compressed configuration when the apparatus is inserted into the prostatic urethra of the patient; and allowing the second member to expand relative to the second member after the apparatus is inserted into the prostatic urethra of the patient, where an outer surface of the second member presses against an inner surface of the prostatic urethra as the second member expands.

The following are examples of some of the materials and compositions described in WO 2016/178829 A1 which may be used with features as described herein. These are merely examples and should not be considered as limiting.

A hemostatic formulation may comprise carboxymethyl chitosan in an amount of about 45% to about 95% by weight, and methyl cellulose in an amount of about 4% to about 12% by weight. In one embodiment, the formulation further comprises hydroxy ethyl cellulose. In one embodiment, the hydroxy ethyl cellulose is in an amount of about 5% to about 15% by weight. In one embodiment, the hydroxy ethyl cellulose is in an amount of about 10% to about 15% by weight. In one embodiment, the hydroxy ethyl cellulose is in an amount of about 12% by weight. In one embodiment, the formulation further comprises calcium alginate. In one embodiment, the calcium alginate is in an amount of about 10% or less by weight. In one embodiment, the calcium alginate is in an amount of about 2% to about 6% by weight. In one embodiment, the calcium alginate is in an amount of about 5% by weight. In one embodiment, the formulation further comprises a polyacrylate. In one embodiment, the polyacrylate may be sodium polyacrylate, potassium polyacrylate, ammonium polyacrylate, monoethanolamine polyacrylate, diethanolamine polyacrylate, or triethanolamine polyacrylate.

A hemostatic formulation may comprise carboxymethyl chitosan in an amount of about 70% to about 80% by weight, and methyl cellulose in an amount of about 4% to about 12% by weight. In one embodiment, the formulation further comprises hydroxy ethyl cellulose. In one embodiment, the hydroxy ethyl cellulose is in an amount of about 5% to about 15% by weight. In one embodiment, the hydroxy ethyl cellulose is in an amount of about 10% to about 15% by weight. In one embodiment, the hydroxy ethyl cellulose is in an amount of about 12% by weight. In one embodiment, the formulation further comprises calcium alginate. In one embodiment, the calcium alginate is in an amount of about 10% or less by weight. In one embodiment, the calcium alginate is in an amount of about 2% to about 6% by weight. In one embodiment, the calcium alginate is in an amount of about 5% by weight. In one embodiment, the formulation further comprises a polyacrylate. In one embodiment, the polyacrylate may be sodium polyacrylate, potassium polyacrylate, ammonium polyacrylate, monoethanolamine polyacrylate, diethanolamine polyacrylate, or triethanolamine polyacrylate.

A hemostatic formulation may comprise carboxymethyl chitosan in an amount of about 70% to about 80% by weight, and methyl cellulose in an amount of about 5% to about 10% by weight. In one embodiment, the formulation further comprises hydroxy ethyl cellulose. In one embodiment, the hydroxy ethyl cellulose is in an amount of about 5% to about 15% by weight. In one embodiment, the hydroxy ethyl cellulose is in an amount of about 10% to about 15% by weight. In one embodiment, the hydroxy ethyl cellulose is in an amount of about 12% by weight. In one embodiment, the formulation further comprises calcium alginate. In one embodiment, the calcium alginate is in an amount of about 10% or less by weight. In one embodiment, the calcium alginate is in an amount of about 2% to about 6% by weight. In one embodiment, the calcium alginate is in an amount of about 5% by weight. In one embodiment, the formulation further comprises a polyacrylate. In one embodiment, the polyacrylate may be sodium polyacrylate, potassium polyacrylate, ammonium polyacrylate, monoethanolamine polyacrylate, diethanolamine polyacrylate, or triethanolamine polyacrylate.

A hemostatic formulation may comprise carboxymethyl chitosan in an amount of about 45% to about 95% by weight, methyl cellulose in an amount of about 4% to about 12% by weight, hydroxy ethyl cellulose in an amount of about 5% to about 15% by weight, and calcium alginate in an amount of about 10% or less by weight.

A hemostatic formulation may comprise carboxymethyl chitosan in an amount of about 70% to about 80% by weight, methyl cellulose in an amount of about 4% to about 12% by weight, hydroxy ethyl cellulose in an amount of about 5% to about 15% by weight, and calcium alginate in an amount of about 10% or less by weight.

A hemostatic formulation may comprise carboxymethyl chitosan in an amount of about 70% to about 80% by weight, methyl cellulose in an amount of about 5% to about 10% by weight, hydroxy ethyl cellulose in an amount of about 5% to about 15% by weight, and calcium alginate in an amount of about 10% or less by weight.

A hemostatic formulation may comprise carboxymethyl chitosan in an amount of about 70% to about 80% by weight, methyl cellulose in an amount of about 5% to about 10% by weight, hydroxy ethyl cellulose in an amount of about 10% to about 15% by weight, and calcium alginate in an amount of about 10% or less by weight.

A hemostatic formulation may comprise carboxymethyl chitosan in an amount of about 70% to about 80% by weight, methyl cellulose in an amount of about 5% to about 10% by weight, hydroxy ethyl cellulose in an amount of about 10% to about 15% by weight, and calcium alginate in an amount of about 2% to about 6% by weight.

A hemostatic formulation may consist essentially of carboxymethyl chitosan in an amount of about 45% to about 95% by weight, methyl cellulose in an amount of about 4% to about 12% by weight, hydroxy ethyl cellulose in an amount of about 5% to about 15% by weight, and calcium alginate in an amount of about 10% or less by weight.

A hemostatic formulation may consist essentially of carboxymethyl chitosan an amount of about 70% to about 80% by weight, methyl cellulose in an amount of about 4% to about 12% by weight, hydroxy ethyl cellulose in an amount of about 5% to about 15% by weight, and calcium alginate in an amount of about 10% or less by weight.

A hemostatic formulation may comprise carboxymethyl chitosan in an amount of about 45% to about 95% by weight, methyl cellulose in an amount of about 4% to about 12% by weight, hydroxy ethyl cellulose in an amount of about 5% to about 15% by weight, calcium alginate in an amount of about 10% or less by weight, and sodium polyacrylate is in an amount of 10% or less by weight.

A hemostatic formulation may consist essentially of carboxymethyl chitosan in an amount of about 70% to about 80% by weight, methyl cellulose in an amount of about 5% to about 10% by weight, hydroxy ethyl cellulose in an amount of about 5% to about 15% by weight, and calcium alginate in an amount of about 10% or less by weight.

A hemostatic formulation may consist essentially of carboxymethyl chitosan in an amount of about 70% to about 80% by weight, methyl cellulose in an amount of about 5% to about 10% by weight, hydroxy ethyl cellulose in an amount of about 10% to about 15% by weight, and calcium alginate in an amount of about 10% or less by weight.

A hemostatic formulation may consisting essentially of carboxymethyl chitosan in an amount of about 70% to about 80% by weight, methyl cellulose in an amount of about 5% to about 10% by weight, hydroxy ethyl cellulose in an amount of about 10% to about 15% by weight, and calcium alginate in an amount of about 2% to about 6% by weight.

A hemostatic formulation may consist essentially of carboxymethyl chitosan in an amount of about 76% by weight, methyl cellulose in an amount of about 10% by weight, hydroxy ethyl cellulose in an amount of about 12% by weight, and calcium alginate in an amount of about 5% by weight.

A hemostatic sponge may comprise carboxymethyl chitosan in an amount of about 45% to about 95% by weight, and methyl cellulose in an amount of about 4% to about 12% by weight. In one embodiment, the sponge further comprises hydroxy ethyl cellulose. In one embodiment, the hydroxy ethyl cellulose is in an amount of about 5% to about 15% by weight. In one embodiment, the hydroxy ethyl cellulose is in an amount of about 10% to about 15% by weight. In one embodiment, the hydroxy ethyl cellulose is in an amount of about 12% by weight. In one embodiment, the sponge further comprises calcium alginate. In one embodiment, the calcium alginate is in an amount of about 10% or less by weight. In one embodiment, the calcium alginate is in an amount of about 2% to about 6% by weight. In one embodiment, the calcium alginate is in an amount of about 5% by weight. In one embodiment, the sponge further comprises a polyacrylate. In one embodiment, the polyacrylate may be sodium polyacrylate, potassium polyacrylate, ammonium polyacrylate, monoethanolamine polyacrylate, diethanolamine polyacrylate, or triethanolamine polyacrylate.

In certain embodiments, the hemostatic sponge has a vertical expansion ratio of 2 or more. In certain embodiments, the hemostatic sponge has a bench degradation rate of less than 20 days. In certain embodiments, the hemostatic sponge has a vertical expansion ratio of 2 or more and a bench degradation rate of less than 20 days. In certain embodiments, the hemostatic sponge has a pliability of over 70 degrees, or over 90 degrees, or over 120 degrees or over 150 degree for example.

A hemostatic sponge may comprise carboxymethyl chitosan in an amount of about 70% to about 80% by weight, and methyl cellulose in an amount of about 4% to about 12% by weight. In one embodiment, the sponge further comprises hydroxy ethyl cellulose. In one embodiment, the hydroxy ethyl cellulose is in an amount of about 5% to about 15% by weight. In one embodiment, the hydroxy ethyl cellulose is in an amount of about 10% to about 15% by weight. In one embodiment, the hydroxy ethyl cellulose is in an amount of about 12% by weight. In one embodiment, the sponge further comprises calcium alginate. In one embodiment, the calcium alginate is in an amount of about 10% or less by weight. In one embodiment, the calcium alginate is in an amount of about 2% to about 6% by weight. In one embodiment, the calcium alginate is in an amount of about 5% by weight. In one embodiment, the hemostatic sponge further comprises sodium polyacrylate. In one embodiment, the sodium polyacrylate is in an amount of 10% or less by weight.

A hemostatic sponge may comprise carboxymethyl chitosan in an amount of about 70% to about 80% by weight, and methyl cellulose in an amount of about 5% to about 10% by weight. In one embodiment, the sponge further comprises hydroxy ethyl cellulose. In one embodiment, the hydroxy ethyl cellulose is in an amount of about 5% to about 15% by weight. In one embodiment, the hydroxy ethyl cellulose is in an amount of about 10% to about 15% by weight. In one embodiment, the hydroxy ethyl cellulose is in an amount of about 12% by weight. In one embodiment, the sponge further comprises calcium alginate. In one embodiment, the calcium alginate is in an amount of about 10% or less by weight. In one embodiment, the calcium alginate is in an amount of about 2% to about 6% by weight. In one embodiment, the calcium alginate is in an amount of about 5% by weight. In one embodiment, the hemostatic sponge further comprises sodium polyacrylate. In one embodiment, the sodium polyacrylate is in an amount of 10% or less by weight.

A hemostatic sponge may comprise carboxymethyl chitosan in an amount of about 45% to about 95% by weight, methyl cellulose in an amount of about 4% to about 12% by weight, hydroxy ethyl cellulose in an amount of about 5% to about 15% by weight, and calcium alginate an amount of about 10% or less by weight.

A hemostatic sponge may comprise carboxymethyl chitosan in an amount of about 70% to about 80% by weight, methyl cellulose in an amount of about 4% to about 12% by weight, hydroxy ethyl cellulose in an amount of about 5% to about 15% by weight, and calcium alginate in an amount of about 10% or less by weight.

A hemostatic sponge may comprise carboxymethyl chitosan in an amount of about 70% to about 80% by weight, methyl cellulose in an amount of about 5% to about 10% by weight, hydroxy ethyl cellulose in an amount of about 5% to about 15% by weight, and calcium alginate in an amount of about 10% or less by weight. In certain embodiments, the hemostatic sponge has a vertical expansion ratio of 2 or more.

A hemostatic sponge may comprise carboxymethyl chitosan in an amount of about 70% to about 80% by weight, methyl cellulose in an amount of about 5% to about 10% by weight, hydroxy ethyl cellulose in an amount of about 10% to about 15% by weight, and calcium alginate in an amount of about 10% or less by weight.

A hemostatic sponge may comprise carboxymethyl chitosan in an amount of about 70% to about 80% by weight, methyl cellulose in an amount of about 5% to about 10% by weight, hydroxy ethyl cellulose in an amount of about 10% to about 15% by weight, and calcium alginate in an amount of about 2% to about 6% by weight.

A hemostatic sponge may comprise carboxymethyl chitosan in an amount of about 45% to about 95% by weight, methyl cellulose in an amount of about 4% to about 12% by weight, hydroxy ethyl cellulose in an amount of about 5% to about 15% by weight, calcium alginate an amount of about 10% or less by weight, and sodium polyacrylate is in an amount of 10% or less by weight.

A hemostatic sponge may comprise essentially of carboxymethyl chitosan in an amount of about 45% to about 95% by weight, methyl cellulose in an amount of about 4% to about 12% by weight, hydroxy ethyl cellulose in an amount of about 5% to about 15% by weight, and calcium alginate in an amount of about 10% or less by weight. In certain embodiments, the hemostatic sponge has a vertical expansion ratio of 2 or more.

A hemostatic sponge may consist essentially of carboxymethyl chitosan in an amount of about 70% to about 80% by weight, methyl cellulose in an amount of about 4% to about 12% by weight, hydroxy ethyl cellulose in an amount of about 5% to about 15% by weight, and calcium alginate in an amount of about 10% or less by weight.

A hemostatic sponge may consist essentially of carboxymethyl chitosan in an amount of about 70% to about 80% by weight, methyl cellulose in an amount of about 5% to about 10% by weight, hydroxy ethyl cellulose in an amount of about 5% to about 15% by weight, and calcium alginate in an amount of about 10% or less by weight.

A hemostatic sponge may consist essentially of carboxymethyl chitosan in an amount of about 70% to about 80% by weight, methyl cellulose in an amount of about 5% to about 10% by weight, hydroxy ethyl cellulose in an amount of about 10% to about 15% by weight, and calcium alginate in an amount of about 10% or less by weight.

A hemostatic sponge may consist essentially of carboxymethyl chitosan in an amount of about 70% to about 80% by weight, methyl cellulose in an amount of about 5% to about 10% by weight, hydroxy ethyl cellulose in an amount of about 10% to about 15%, and calcium alginate in an amount of about 2% to about 6% by weight.

A hemostatic sponge may consist essentially of carboxymethyl chitosan in an amount of about 76% by weight, methyl cellulose in an amount of about 10% by weight, hydroxy ethyl cellulose in an amount of about 12% by weight, and calcium alginate in an amount of about 5% by weight.

A hemostatic sponge may have a porosity of at least 20%. A hemostatic sponge may have a porosity of at least 30%. A hemostatic sponge may have a porosity of at least 40%.

The hemostatic formulation or sponge may further comprise a binding agent, a clotting accelerator, a therapeutic agent, or a combination thereof, or a mixture thereof. A binding agent may be dissolved with the individual components in a solvent. A binding agent may further increase or decrease the flexibility of sponge, the liquid holding capacity of sponge, and/or the rate at which sponge absorbs liquid. Binding agents may be soluble in water and/or other solvents. In some embodiments, the hemostatic formulation or sponge may comprise a single binding agent or a combination of different binding agents. In some embodiments, the hemostatic formulation or sponge may not comprise any binding agents. In such embodiments, the individual components may adhere together without a binding agent.

The hemostatic formulation or sponge may comprise a clotting accelerator to speed the clotting process. A clotting accelerator may be dissolved with the individual components in a solvent. The amount of clotting accelerator added to the sponge formulation may depend upon the application, but it may be a smaller percentage by weight or a larger percentage by weight as compared to the individual components of the formulation or the sponge. The hemostatic formulation or sponge may comprise a single clotting accelerator or a combination of different clotting accelerators. In some embodiments, such as where the individual components are sufficient to clot blood by itself, the hemostatic formulation or sponge may not comprise any clotting accelerators.

The hemostatic formulation or sponge may further comprise one or more therapeutic agents. The one or more therapeutic agents may include anti-inflammatory agents, antibiotics, antiviral agents, antifungals, antiprotozoal agents, immunosuppressive agents, other suitable drugs, or combinations thereof, or mixtures thereof. The one or more therapeutic agents may be mixed with the hemostatic sponge formulation while the sponge is being made or may be applied to a surface of the sponge after manufacture.

It should be understood that the foregoing description is only illustrative. Various alternatives and modifications can be devised by those skilled in the art. For example, features recited in the various dependent claims could be combined with each other in any suitable combination (s). In addition, features from different embodiments described above could be selectively combined into a new embodiment. Accordingly, the description is intended to embrace all such alternatives, modifications and variances with the invention being defined by the appended claims.

## Claims

1. An apparatus comprising:
a first layer (12), where the first layer (12) comprises a scaffold structure forming an inner lumen (16) along a length of the scaffold structure, and where the first layer (12) comprises a bioresorbable material; and
a second layer (14) on the first layer (12), where the second layer (14) comprises a bioresorbable material, where the second layer (14) surrounds a majority of the first layer (12), wherein the second layer (14) is configured to hydroscopicly swell; and
the scaffold structure has a distal end (20) which extends past a distal (26) end of the second layer;
**characterized in that** the scaffold structure comprises protruding anchors (44) which extend into the second layer (14) and are sized and shaped to fix the scaffold structure to tissue of a patient after the second layer (14) is resorbed or dissolved inside the patient.

2. An apparatus as in claim 1 where the scaffold structure has a general lattice shape including at a tip (22) of a distal end (20) of the scaffold structure, and where the second layer (14) comprises Chitosan.

3. An apparatus as in claim 1 where at least one portion of the scaffold structure is configured to expand from a collapsed configuration to an expanded configuration inside a patient.

4. An apparatus as in claim 1 where the second layer (14) is configured to hydroscopicly swell into a shape having a enlarged distal end configured to be located against a bladder neck of a patient.

5. An apparatus as in claim 4 where the second layer (14) is configured to hydroscopicly swell into the shape with an outer conical shape extending from the enlarged distal end in a direction towards a proximal end of the second layer.

6. An apparatus as in claim 1 where at least one portion of the second layer (14) has an outer diameter which is configured to hydroscopicly swell between about 200-500 percent from a non-swelled first configured to a hydroscopicly swelled second configuration.

7. An apparatus as in claim 1 further comprising a protective cover on the second layer to limit moisture entering the second layer, where the protective cover is configured to be slid off of the second layer after the apparatus is inserted into patient.

8. An apparatus as in claim 1 where the first layer (12) has a first degradation rate which is different than a second degradation rate of the second layer (14).

9. An apparatus as in claim 1 where the first layer comprises a first member (12), where the second layer comprises a second member (14), where the second member (14) is compressible on the first member (12), and where the second member (14) is configured to resiliently expand on the first member (12) from a compressed configuration relative to the first member (12) to an expanded configuration relative to the first member (12).

10. An apparatus as in claim 1 where the apparatus is configured to be inserted into a prostatic urethra of a patient, where the second layer (14) is configured to expand relative to the first layer (12) after the apparatus is inserted into the prostatic urethra of the patient, and where an outer surface of the second layer (14) is configured to press against an inner surface of the prostatic urethra as the second member (14) expands.

11. A method comprising:
providing a first member (12), where the first member comprises a scaffold structure forming an inner lumen (16) along a length of the scaffold structure, and where the first member (12) comprises a bioresorbable material;
providing a second member (14) on the first member (12), where the second member (14) comprises a bioresorbable material, where the second layer surrounds at least a portion of the first member (12), and where the second member (14) is configured to hydroscopicly swell; and
the first member has a distal end (20) which extends past a distal (26) end of the second member;
**characterized in that** the first (12) member comprises protruding anchors (44) which extend into the second member (14) and are sized and shaped to fix the first member (12) to tissue of a patient after the second member (14) is resorbed or dissolved inside the patient.

## Patentansprüche

1. Vorrichtung, umfassend:
eine erste Schicht (12), wobei die erste Schicht (12) eine Gerüststruktur umfasst, die entlang einer Länge der Gerüststruktur ein inneres Lumen (16) bildet, und wobei die erste Schicht (12) ein bioresorbierbares Material umfasst; und
eine zweite Schicht (14) auf der ersten Schicht (12), wobei die zweite Schicht (14) ein bioresorbierbares Material umfasst, wobei die zweite Schicht (14) einen Großteil der ersten Schicht (12) umgibt, wobei die zweite Schicht (14) so konfiguriert ist, dass sie hygroskopisch quillt; und
die Gerüststruktur ein distales Ende (20) aufweist, das sich über ein distales (26) Ende der zweiten Schicht hinaus erstreckt;
**dadurch gekennzeichnet, dass** die Gerüststruktur vorstehende Verankerungen (44) umfasst, die sich in die zweite Schicht (14) erstrecken und so dimensioniert und geformt sind, dass sie die Gerüststruktur an Gewebe eines Patienten befestigen, nachdem die zweite Schicht (14) im Patienten resorbiert oder aufgelöst wurde.

2. Vorrichtung nach Anspruch 1, wobei die Gerüststruktur eine allgemeine Gitterform aufweist, einschließlich einer Spitze (22) eines distalen Endes (20) der Gerüststruktur, und wobei die zweite Schicht (14) Chitosan umfasst.

3. Vorrichtung nach Anspruch 1, wobei mindestens ein Teil der Gerüststruktur so konfiguriert ist, dass er sich innerhalb eines Patienten von einer zusammengefalteten Konfiguration zu einer expandierten Konfiguration ausdehnt.

4. Vorrichtung nach Anspruch 1, wobei die zweite Schicht (14) so konfiguriert ist, dass sie hygroskopisch zu einer Form aufquillt, die ein vergrößertes distales Ende aufweist, das so konfiguriert ist, dass es an einem Blasenhals eines Patienten anliegt.

5. Vorrichtung nach Anspruch 4, wobei die zweite Schicht (14) so konfiguriert ist, dass sie hygroskopisch zu einer Form mit einer äußeren konischen Form aufquillt, die sich vom vergrößerten distalen Ende in Richtung eines proximalen Endes der zweiten Schicht erstreckt.

6. Vorrichtung nach Anspruch 1, wobei mindestens ein Abschnitt der zweiten Schicht (14) einen Außendurchmesser aufweist, der so konfiguriert ist, dass er zwischen etwa 200 und 500 Prozent von einer nicht gequollenen ersten Konfiguration zu einer hygroskopisch gequollenen zweiten Konfiguration quillt.

7. Vorrichtung nach Anspruch 1, die ferner eine Schutzabdeckung auf der zweiten Schicht umfasst, um das Eindringen von Feuchtigkeit in die zweite Schicht zu begrenzen, wobei die Schutzabdeckung so konfiguriert ist, dass sie von der zweiten Schicht abgezogen werden kann, nachdem die Vorrichtung in den Patienten eingeführt wurde.

8. Vorrichtung nach Anspruch 1, wobei die erste Schicht (12) eine erste Abbaurate aufweist, die sich von einer zweiten Abbaurate der zweiten Schicht (14) unterscheidet.

9. Vorrichtung nach Anspruch 1, wobei die erste Schicht ein erstes Element (12) umfasst, wobei die zweite Schicht ein zweites Element (14) umfasst, wobei das zweite Element (14) auf dem ersten Element (12) komprimierbar ist und wobei das zweite Element (14) so konfiguriert ist, dass es sich auf dem ersten Element (12) aus einer komprimierten Konfiguration relativ zum ersten Element (12) in eine expandierte Konfiguration relativ zum ersten Element (12) elastisch ausdehnt.

10. Vorrichtung nach Anspruch 1, wobei die Vorrichtung so konfiguriert ist, dass sie in die Prostata-Harnröhre eines Patienten eingeführt werden kann, wobei die zweite Schicht (14) so konfiguriert ist, dass sie sich relativ zur ersten Schicht (12) ausdehnen kann, nachdem die Vorrichtung in die Prostata-Harnröhre des Patienten eingeführt wurde, und wobei eine Außenfläche der zweiten Schicht (14) so konfiguriert ist, dass sie gegen eine Innenfläche der Prostata-Harnröhre drückt, wenn sich das zweite Element (14) ausdehnt.

11. Verfahren, umfassend:
Bereitstellen eines ersten Elements (12), wobei das erste Element eine Gerüststruktur umfasst, die entlang einer Länge der Gerüststruktur ein inneres Lumen (16) bildet, und wobei das erste Element (12) ein bioresorbierbares Material umfasst;
Bereitstellen eines zweiten Elements (14) auf dem ersten Element (12), wobei das zweite Element (14) ein bioresorbierbares Material umfasst, wobei die zweite Schicht mindestens einen Teil des ersten Elements (12) umgibt und wobei das zweite Element (14) so konfiguriert ist, dass es hygroskopisch quillt; und
wobei das erste Element ein distales Ende (20) aufweist, das sich über ein distales Ende (26) des zweiten Elements hinaus erstreckt;
**dadurch gekennzeichnet, dass** das erste Element (12) vorstehende Verankerungen (44) umfasst, die sich in das zweite Element (14) erstrecken und so dimensioniert und geformt sind, dass sie das erste Element (12) am Gewebe eines Patienten fixieren, nachdem das zweite Element (14) im Patienten resorbiert oder aufgelöst wurde.

## Revendications

1. Appareil comprenant :
une première couche (12), où la première couche (12) comprend une structure d'échafaudage formant une lumière interne (16) sur toute la longueur de la structure d'échafaudage, et où la première couche (12) comprend un matériau biorésorbable ; et
une deuxième couche (14) sur la première couche (12), la deuxième couche (14) comprenant un matériau biorésorbable, la deuxième couche (14) entourant la majeure partie de la première couche (12), la deuxième couche (14) étant configurée pour gonfler par hygroscopie ; et
la structure d'échafaudage ayant une extrémité distale (20) qui s'étend au-delà d'une extrémité distale (26) de la deuxième couche ;
**caractérisée en ce que** la structure d'échafaudage comprend des ancrages saillants (44) qui s'étendent dans la deuxième couche (14) et sont dimensionnés et formés pour fixer la structure d'échafaudage au tissu d'un patient après que la deuxième couche (14) a été résorbée ou dissoute à l'intérieur du patient.

2. Appareil selon la revendication 1, dans lequel la structure d'échafaudage a une forme générale en treillis comprenant une pointe (22) à une extrémité distale (20) de la structure d'échafaudage, et dans lequel la deuxième couche (14) comprend du chitosane.

3. Appareil selon la revendication 1, dans lequel au moins une partie de la structure d'échafaudage est configurée pour se déployer d'une configuration repliée à une configuration déployée à l'intérieur d'un patient.

4. Appareil selon la revendication 1, dans lequel la deuxième couche (14) est configurée pour gonfler par hygroscopie en une forme ayant une extrémité distale élargie configurée pour être placée contre le col vésical d'un patient.

5. Appareil selon la revendication 4, dans lequel la deuxième couche (14) est configurée pour gonfler par hygroscopie en une forme avec une forme conique extérieure s'étendant depuis l'extrémité distale élargie dans une direction vers une extrémité proximale de la deuxième couche.

6. Appareil selon la revendication 1, dans lequel au moins une partie de la deuxième couche (14) a un diamètre extérieur qui est configuré pour gonfler par hygroscopie entre environ 200 et 500 % par rapport à une première configuration non gonflée vers une deuxième configuration gonflée par hygroscopie.

7. Appareil selon la revendication 1, comprenant en outre un couvercle protecteur sur la deuxième couche pour limiter l'entrée d'humidité dans la deuxième couche, le couvercle protecteur étant configuré pour être retiré de la deuxième couche après l'insertion de l'appareil dans le patient.

8. Appareil selon la revendication 1, dans lequel la première couche (12) a un premier taux de dégradation qui est différent d'un deuxième taux de dégradation de la deuxième couche (14).

9. Appareil selon la revendication 1, dans lequel la première couche comprend un premier élément (12), la deuxième couche comprend un deuxième élément (14), le deuxième élément (14) est compressible sur le premier élément (12), et où le deuxième élément (14) est configuré pour se dilater de manière élastique sur le premier élément (12) depuis une configuration comprimée par rapport au premier élément (12) jusqu'à une configuration dilatée par rapport au premier élément (12).

10. Appareil selon la revendication 1, dans lequel l'appareil est configuré pour être inséré dans l'urètre prostatique d'un patient, dans lequel la deuxième couche (14) est configurée pour se dilater par rapport à la première couche (12) après que l'appareil a été inséré dans l'urètre prostatique du patient, et dans lequel une surface extérieure de la deuxième couche (14) est configurée pour appuyer contre une surface intérieure de l'urètre prostatique lorsque le deuxième élément (14) se dilate.

11. Procédé comprenant :
la fourniture d'un premier élément (12), dans lequel le premier élément comprend une structure d'échafaudage formant une lumière interne (16) sur la longueur de la structure d'échafaudage, et dans lequel le premier élément (12) comprend un matériau biorésorbable ;
la fourniture d'un deuxième élément (14) sur le premier élément (12), le deuxième élément (14) comprenant un matériau biorésorbable, la deuxième couche entourant au moins une partie du premier élément (12), et le deuxième élément (14) étant configuré pour gonfler par hygroscopie ; et
le premier élément a une extrémité distale (20) qui s'étend au-delà d'une extrémité distale (26) du deuxième élément ;
**caractérisé en ce que** le premier élément (12) comprend des ancrages saillants (44) qui s'étendent dans le deuxième élément (14) et sont dimensionnés et formés pour fixer le premier élément (12) au tissu d'un patient après que le deuxième élément (14) a été résorbé ou dissous à l'intérieur du patient.
